# EUROPEAN PATENT APPLICATION

(11) **EP 2 564 877 A1**
(43) Date of publication of application: **06.03.2013**
(21) Application number: 12176492.2
(22) Date of filing: 16.07.2012
(51) Int. Cl.: A61L 9/12

(54) **An improvement of a deodorant device that gradually releases fragrance**

(30) Priority: 29.08.2011 IT PI20110093
(71) Applicant: Artoni, Simone, 42015 Correggio (RE) (IT)
(72) Inventor: Artoni, Simone, 42015 Correggio (RE) (IT)
(74) Representative: Turini, Laura

(57) **Abstract**

The present invention concerns an ambiance perfume device (1) comprising:
- A small bottle (2) for containing a perfuming liquid and provided with an opening (4);
- A perfume diffuser element (30) connected to the small bottle (2) in such a way as to come into contact with the liquid contained in the small bottle through said opening (4), said diffuser element being further realized in a material suitable for absorbing the perfuming liquid in such a way as to absorb it and gradually release it in the ambiance;

In accordance with the invention, a protective screen (5) is further included in a non-absorbing material for liquids and arranged below the diffuser element (30) in such a way that, in use, the eventual drops of perfuming liquid released by the diffuser element (30) fall on the protective screen. In addition, the perfume diffuser element (30) includes a blind channel (31), the perfume diffuser element being further arranged with said channel (31) substantially in axis with the opening (4) in such a way that in correspondence of the overturning of the container (2) at least a part of the liquid contained in the container (2) is collected inside the channel (31).

## Description

### Technical field

The present invention refers to the technical field relative to the deodorant devices that gradually release fragrance.

In particular, the invention refers to an improvement of a deodorant device formed by a small bottle for the containment of the fragrance liquid and by an upper part provided with a diffuser element of the liquid of an absorbing material. The improvement is such as to allow both to block the accidental fall of drops of liquid that can eventually be expelled from the diffuser element during the functioning and to optimize the diffusion of the fragrance.

### Background art

Utility model application PI2002U000021, filed on 15/10/2002 in the name of GOLDEN PELL under the title "Gradual transmission fragrance system for small rooms", has been public for a long time.

Said utility model describes an ambiance perfume device structurally formed by a small bottle for containing a perfume diffuser liquid and by a wooden top screwable to the neck of the small bottle. Interposed between the top and the mouth of the small bottle there is a disk of cellulose that absorbs the liquid, thus dosing in time the absorption of said liquid by the overlying wooden top. In this way, by overturning for a few seconds said device, the disk becomes impregnated with perfume, gradually releasing it in the wooden top and from it towards the external ambiance. This simple system allowed a gradual release that was more or less constant in time until the exhaustion of the product contained in the small bottle, therefore avoiding large diffusions at the beginning of use, becoming always more gradual towards the end of the use.

Such a solution has the advantage of using a diffuser element that does not work by immersion (i.e. it is not in contact by one of its ends with the liquid contained in the small bottle) so that in order to diffuse the fragrance it has to be overturned. Nevertheless, also this device is not exempt from various technical inconveniences.

Above all, inevitably, during use, the wood increments the quantity of liquid perfume of which it results soaked to such a point that it can drip and ruin the surfaces on which the device itself is placed.

Moreover, it is known how the liquid inflates the wood, ruining for such a reason the thread and thus rendering difficult, and at times even impossible, the removal and the re-positioning of the top of the small bottle, in case it becomes necessary to do so.

Some solutions have been presented in order to solve the technical problem of the dripping.

For example, in US4742960 a wick made of fabric is described which however works by immersion into the liquid contained in the small bottle. The wick results hooked to a lifting system that passes outside the small bottle through a slip found in the sealing top. By pulling upwards the support the wick is lifted in part outside the small bottle, releasing the essence of which it is soaked and maintaining its lower end always in contact with the liquid contained in the small bottle. The underlying top collects the eventual drops that fall from the wick.

This solution is however by immersion, i.e. it works in a completely opposed way with respect to the solution by overturning, wherein the diffuser element comes into contact with the liquid only if the small bottle is overturned. This solution at immersion, being the wick in continuous contact with the liquid of the small bottle, creates a technical inconvenience by which it is not possible to dose the diffusion, and instead it will always take place in a continuative manner up to when the level of the liquid contained will be almost exhausted, obviously consuming an excessive quantity of fragrance. Basically, the fragrance element, as it always happens in the devices by immersion, has such a length as to reach the base of the small bottle so as to be able to absorb also the minimum level of liquid contained. Above all, the continuous contact with the liquid damages the wick itself or the diffuser element used.

On the other hand, in an overturning solution, it is sometimes necessary to overturn and maintain the device overturned for a certain pre-established period of time so that the diffuser element, mainly in the first use cycles, becomes sufficiently impregnated with the liquid in such a way as to diffuse the fragrance in an efficient way.

### Disclosure of invention

It is therefore the aim of the present invention to provide a new type of perfume device for ambiances that solves at least in part these and other technical problems.

In particular, it is the aim of the present invention to provide a perfume device for ambiances configured in such a way that, even if the diffuser element of the perfume can let drops of liquid fall, said drops are impeded from sliding or falling along the perfume device itself, thus eliminating the risk of staining the underlying surfaces on which the perfume device results placed.

It is also the aim of the present invention to provide a perfume device 1 wherein the fragrance diffuser element, although being arranged in the perfume device in such a way as not to work by immersion, allows an efficient impregnation to diffuse the fragrance since the first use cycles and, in case of excessive impregnation during use, to prevent that the dropping slides along the device itself.

These and other aims are therefore reached with the present perfume device 1 for ambiances, in accordance with claim 1.

In accordance with the invention, the perfume device (1) for ambiance comprising:
- A container (2) for the containment of a perfuming liquid and provided with an opening (4) for the passage of the liquid;
- A diffuser element (30) of the perfume connected above the container (2) and configured in such a way that it can come into contact with the liquid contained in the container through said opening (4) in correspondence of an overturning of the container (2), the diffuser element being further realized in a material permeable to the perfuming liquid in such a way as to absorb it and gradually release it in the ambiance;
and wherein a protective screen (5) is further included, impermeable to liquids and interposed between the diffuser element (30) and the container (2) in such a way that, in use, the eventual drops of perfuming liquid released by the diffuser element (30) fall on said protective screen.

In accordance with the invention, the perfume diffuser element (30) also includes a blind channel (31). The perfume diffuser element is therefore arranged with said channel (31) substantially in axis with the opening (4) in such a way that in correspondence of the overturning of the container (2) at least a part of the liquid contained in the container (2) is collected inside the channel (31).

In this way, said technical problems are easily solved. In particular, all the drops eventually released by the diffuser 30 are collected by the protective screen and are thus impeded from falling on surfaces which could be ruined, such as the dashboard of an automobile when said perfume device is used inside one's own car.

The protective screen can therefore be realized integrally in a non-absorbing material for liquids (therefore imperrneab7.e), such as in plastic or in absorbing materials but covered with a layer that renders it impermeable.

In all cases, thanks to said impermeability, the liquid collected stays on it without the risk of being absorbed again to then drip away. At any time, the liquid collected can be poured away from the perfume device, in particular from the screen that has collected it, therefore avoiding that eventual oscillations of the perfume device itself, for example during its use in a car, can cause its pouring, above all when it is present in large quantities.

In addition, being the device of the overturnable type (i.e. with the diffuser element 30 that does not work by immersion but by overturning), the channel 31 enhances the good absorption of the liquid, above all in the first use cycles, while, precisely in case of excessive absorption, the screen prevents the dripping.

Advantageously, the protective screen can be of a substantially annular shape with a hole (10) that results arranged in axis with the opening (4) of the container (2).

Advantageously, the protective screen (5) can be of such a type as to result applicable to the container (2) in a removable way.

For example, advantageously, the container (2) can be provided with a threaded neck (3) finishing with said opening (4) and the protective screen (5) can form a receiving channel (8) in which the threaded neck (3) of the container is inserted. The receiving channel (8) is in turn provided with said hole (10) and with a complementary thread (11) in such a way that the protective screen (5) results screwable directly on the neck (3) of the container and with said openings (4, 10) in axis between them.

This solution allows an easy application of the protective screen, which is thus easily removable.

Advantageously, in a possible solution, the protective screen has a disk shape.

Moreover, advantageously, the protective screen can be concave to allow the collection of the liquid.

Advantageously, the protective screen is made of plastic or of a material permeable to liquids, but in this case covered at least partially with an impermeable layer.

Advantageously, the protective screen includes an O-Ring (35) for retaining the collected liquid.

Advantageously, the diffuser element (30) is arranged directly on the protective screen (5).

Advantageously, the diffuser element is, according to the choice:
- Lain on the protective screen (5);
- Configured in such a way as to be fixed on the protective screen (5) with a pre-determined degree of mechanical interference.

In both cases, not being present anymore a thread found in the diffuser element to screw it directly against the mouth of the container, the duration of the diffuser element is extremely improved, allowing its easy removal and repeated applications as well.

Advantageously, a cage-shaped retain element (20) can be included and connected to the protective screen (5) in such a way that the diffuser element is found between the protective screen (5) and said cage (20).

This solution improves the safety in case of an accidental detachment of the diffuser element, not screwed anymore to the bottle neck as per the background art.

Advantageously, the retain element (20) includes one or more hooks (21) at the ends that engage in a detachable manner into respective seats (13) found in the protective screen (5).

Advantageously, the perfume diffuser element (30) is entirely contained above the protective screen (5).

Advantageously, a pin (40) can also be included, having a wide head (41) in such a way as to become blocked in the corresponding hole (10) of the protective screen (5), the pin being holed axially so as to form a passing channel for the passage of the liquid contained in the container.

It is also described here an assembly (5, 30) of perfume diffusion configured to result applicable in a removable manner to a container (2) of perfuming liquid, said assembly comprising a protective screen (5) of annular shape provided with a hole (10) and impermeable to liquids, above which a perfume diffuser element (30) is included and **characterized in that** the perfume diffuser element (30) includes a blind channel (31), the diffuser element being further arranged with said channel (31) substantially in axis with the hole (10) in such a way that, in use, when the assembly is applied to the container (2), in correspondence of the overturning of the container (2), at least a part of the liquid contained in the container (2) is collected inside the channel (31) passing through the hole (10).

This system is advantageous since it can be applied to different rechargeable containers, for example.

Advantageously, said assembly further comprises a cage-shaped retain element (20) connected to the protective screen (5) to retain the diffuser element (30).

### Brief description of drawings

Further characteristics and advantages of the present invention will result clearer with the description that follows of some embodiments, made to illustrate but not to limit, with reference to the annexed drawings, wherein:

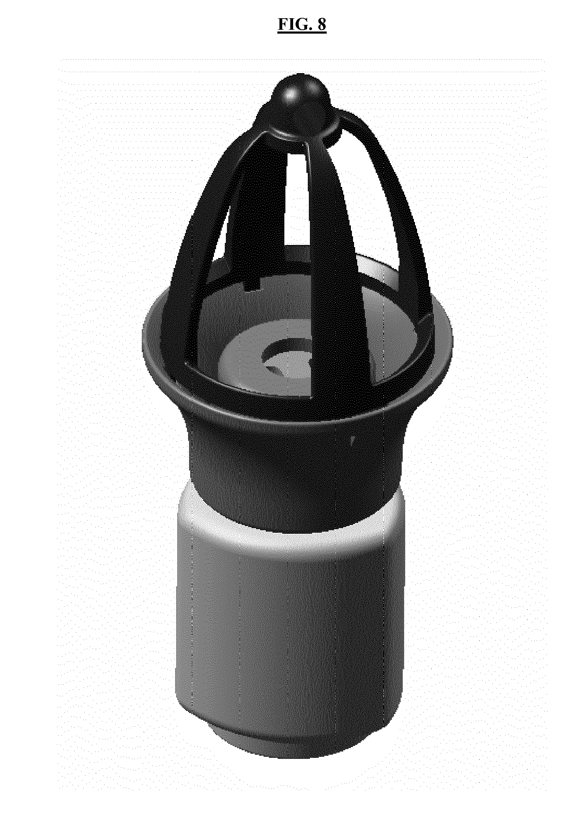

- Figure 1, figure 2 and figure 8 show three axonometric views of the present perfume device in accordance with the invention. Just for clarity purposes, said figures omit the presence of the diffuser element 30;
- Figure 3 shows a transversal section that cuts out the entire perfume device;
- Figure 4 shows in axonometric view the preferred solution relative to the realization of the protective screen 5;
- Figure 5 shows in section a detail of hooking between the cage 20 and the protective screen 5 together with the threads that allow to screw the screen to the neck of the small bottle;
- Figure 6 and figure 7 show two further axonometric views of the perfume device lacking the small bottle.

### Description of some preferred embodiments

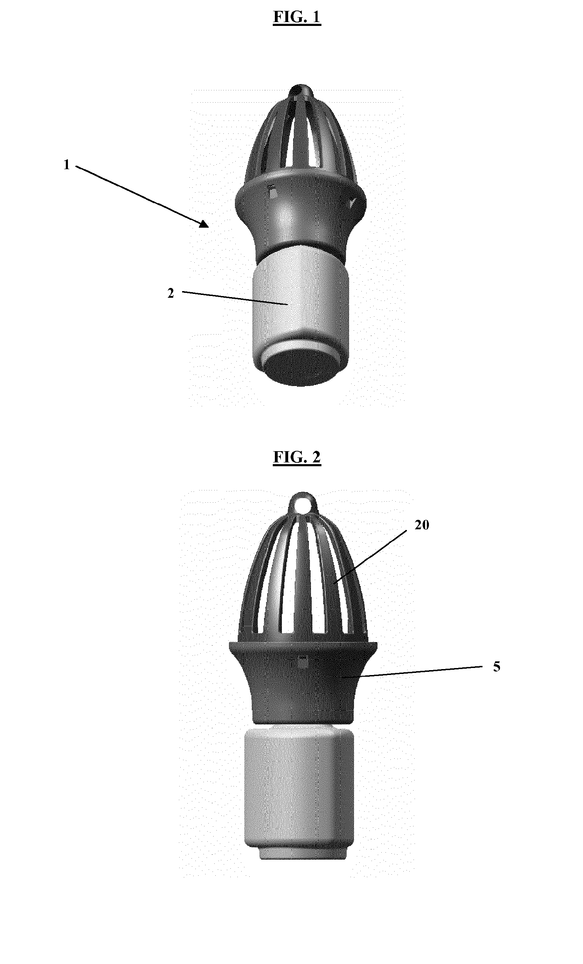

Figure 1 and figure 2 show a perfume device for ambiances (from now on called "perfume device" for simplification purposes) according to the invention.

The perfume device includes a small bottle 2 for the containment of a liquid. The small bottle can obviously be realized in any material (preferably glass or plastic) and can have any size. Nevertheless, due to its application in the field of fragrances for small spaces (such as cars), the small bottle is preferably of small dimensions, i.e. in the order of the 4 to 15 ml for its use in a car. Dimensions in the order of the 50 to 300 ml for the use in domestic rooms are anyway foreseen as well.

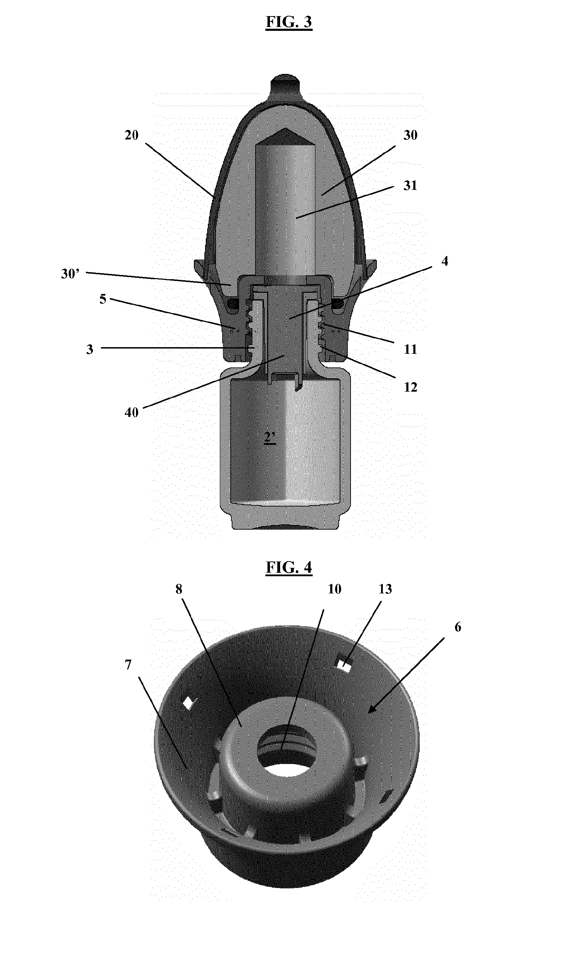

The section of figure 3 shows the small bottle 2 which forms a containment volume 2' into which the perfuming liquid is found. The small bottle, always as shown in the section of figure 3, includes a neck 3 that forms an opening 4 at its end, which is of access to the internal containment volume 2', exactly as an ordinary small bottle of perfume. Always as shown in the section of figure 3, the neck is externally threaded with a thread 12. In this way, according to the invention, in said first embodiment a protective screen 5, in a non-absorbing material for liquids (for example, plastic), can be screwed in correspondence of said thread, as clarified right below.

The axonometric view of figure 4 shows the protective screen 5. It has a concave and circular shape so as to form a volume 6 similar to a cup suitable for collecting liquid. The volume 6 is therefore delimited externally by the circular wall 7 and internally by a cylindrical wall 8 holed axially through a passing hole 10. The cylindrical wall therefore forms a channel open on one side through the hole 10.

As better shown in the section of figure 3, the cylindrical part 8, which is lifted from the base of the volume 6 upwards, serves, in this possible constructive solution, to form said receiving channel in which the neck of the bottle 3 is inserted. In order to realize a stable connection, in said channel 8 a thread 11 is included, which engages on the complementary thread 12 found on the neck of the bottle. In this way, it is possible to easily screw said screen 5 directly to the neck 3 of the bottle with the hole 10 of the screen in axis with the opening 4 of the bottle neck itself.

Always the view of figure 4 shows four cuttings 13 which, as clarified right below, serve as anchor points for a retain element 20 in the shape of a cage-shaped dome that is arranged above the protective screen.

It is obviously clear that, according to the present invention, the shapes of the protective screen can be different from the one described, and structurally even simpler. For example, the screen could also be in the shape of a disk, flat or slightly concave, and it can have a simple axial and central hole provided with a short thread for being screwed to the neck of the bottle.

In all the cases described, it is anyway essential that the protective screen, independently of its shapes, is in a non-absorbing material for liquids. Obviously, plastic or materials such as PVC are easily and economically workable in appropriate moulds and therefore are preferred materials.

Going on with the structural description of the invention, the section of figure 3 shows a perfume diffuser element 30 which is applied above the protective screen 5 in such a way as to come into contact with the liquid contained in the small bottle through the holes 10 and 4 in axis between them. In this way, as per the background art, by simply overturning the perfume device the liquid wets the diffuser element 30, which absorbs the perfume and gradually releases it. In this sense, it is essential that the diffuser element is realized, unlike the protective screen, in a material suitable for absorbing liquids, such as wood or cardboard, pressed cellulose and cotton, cork, pressed fabrics, felt.

In the preferred embodiment of the invention, in which the protective screen is concave, as per the attached figures, the diffuser element 30 presents the lower end 30' shaped so as to be inserted at the basis of the volume 6 with a predetermined degree of mechanical interference. In this way, its application is rapid and simple, and complex connection systems are therefore not requested.

This solution is extremely advantageous since, unlike the background art, it is not necessary anymore to include a thread on the element 30 that is screwed directly to the neck of the bottle and that risks of becoming ruined after time in virtue of the re-inflation due to the liquid. In this way, the diffuser element 30 can be removed and re-inserted many times, continuing to remain perfectly functioning.

Always as shown in the section of figure 3, the diffuser element is holed axially with a blind channel 31 (the section highlights the blind channel 31 found along the longitudinal axis of the element 30). Such a channel 31 has the function of collecting inside it the liquid when the small bottle is overturned in such a way as to realize a larger surface of absorption for the element 30 and that starts from the inside of the element 30 itself. This solution guarantees, above all, a better diffusion of the essence.

In order to guarantee the retain and to avoid completely the risk of detachments of the diffuser element 30 in the overturning phase, the application of the retain element 20 is therefore foreseen.

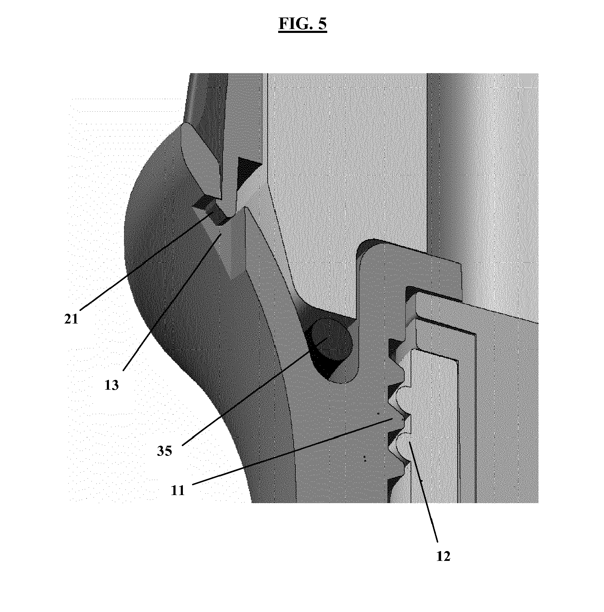

As better shown in the axonometric views of figure 1 and figure 2, such a retain element has the shape of a cage-shaped dome that in fact forms a cage. The ends of such a retain element 20 include connection means that, in a possible solution, have the shape of hooks 21 suitable for engaging in the cuttings 13 found on the wall 7 of the protective screen (see detail of figure 5). As shown in figure 5, an eventual O-Ring 35 can be foreseen at the basis of the screen. The O-Ring has the function of limiting the exit of the liquid from the volume 6 during eventual oscillations of the perfume device (for example, when hung to the rear-view mirror of an automobile).

The retain element also includes, on top of the dome, a ring for the passage of a thread that allows to hang the perfume device to a fixed support, such as the rear-view mirror of a car.

The retain element 20 therefore guarantees the retain of the diffuser device 30 and allows to hang the device to any support in a safe manner.

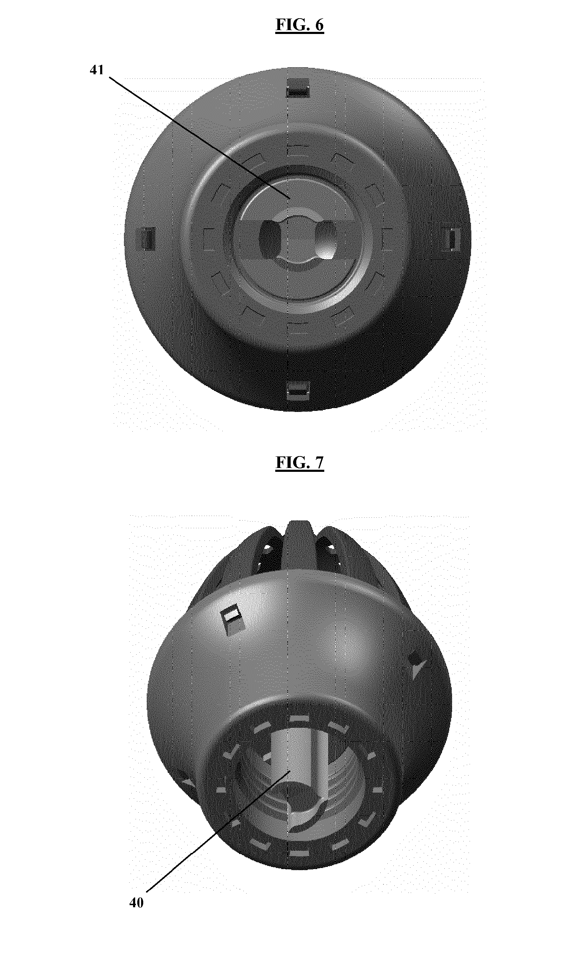

Going back to figure 3 and to the subsequent figures 6 and 7, the presence of a pin 40 is highlighted, having a wide head 41 in such a way as to become blocked in the respective hole 10 of the protective screen 5. The pin is holed axially and forms a passing channel.

In this way, it can be used simply to hole the eventual sealing membrane of a new small bottle. The pin can therefore be removed or left in position, as shown in figure 3.

Such a pin is not anyway essential, since the sealing membrane can be holed using any other system.

Having structurally described the invention, we now pass onto its function description.

The user overturns one or more times the perfume device in such a way that the liquid contained in the small bottle wets the diffuser element 30, passing through the two openings (4, 10) in axis between them (eventually through the pin 40 holed in axis in case it is present). In particular, in the case of a diffuser element having the blind channel 31, the liquid will wet all the channel and it will be thus obtained a greater surface of absorption for the liquid.

The cage 20, during the overturning, impedes the detachment of the diffuser element 30. Nevertheless, the degree of mechanical interference that connects the element 30 to the protective screen 5 can be selected at such a value as to guarantee not only the retain without the need for the cage 20 but even the hanging of the entire device to a fixed support through an eventual hook, completely similar to that of the cage 20, but found directly on the diffuser element.

During use, therefore, the diffuser element 30 soaked of liquid diffuses the fragrance gradually in the ambiance. In case the level of liquid absorbed by the element 30 is high to such an extent that one or more perfume drops are released, said drops will be impeded from falling since they will be collected into the volume 6 formed by the protective screen placed below the diffuser element 30.

A second variant of the invention is completely identical to the preceding one except for the fact that the diffuser element 30 is simply lain on the protective screen without mechanical interference and is retained in position by the single cage-shaped dome 20.

In a third preferred embodiment of the invention, the realization of a small bottle can be thought of that brings integrated to it a disk that serves as a protective screen or includes a simple annular disk that engages by interference along the body of the small bottle.

The various parts, i.e. the small bottle 2, the protective screen 5 furnished with cage and the diffuser element 30, can be produced as spare parts separated between them.

According to the attached figures, it is clear that the diffuser element 30 is configured in such a way as to work by overturning and not by immersion. Its drop-like shape, eventually modifiable in other shapes, is such that it is entirely arranged above the protective screen and it does not include any end that penetrates into the container in such a way that said end can come into contact with the liquid contained.

It is however clear that infinite variants of the shape of the diffuser element could be foreseen, some of which also with an end that penetrates into the container but with a level of liquid and a length of end adjusted in such a way that said end does not work by immersion but, precisely, by overturning.

The device described could however work, even if not in an optimal way, by immersion.

## Claims

1. An ambiance perfume device (1) comprising:
- A container (2) for containing a perfuming liquid and provided with an opening (4) for the passage of the liquid;
- A perfume diffuser element (30) connected above the container (2) and configured in such a way that it can come into contact with the liquid contained in the container through said opening (4) in correspondence of the overturning of the container (2), the diffuser element being further realized in a material permeable to the perfuming liquid so as to absorb it and gradually release it in the ambiance;
and wherein a protective screen (5) is further included that is impermeable to liquids and is interposed between the diffuser element (30) and the container (2) in such a way that, in use, the eventual drops of perfuming liquid released by the diffuser element (30) fall on the protective screen and
**charactarised in that** the perfume diffuser element (30) foresees a blind channel (31), the perfume diffuser element being further arranged with said channel (31) substantially in axis with the opening (4) in such a way that in correspondence of the overturning of the container (2) at least a part of the liquid contained in the container (2) is collected inside the channel (31).

2. A perfume device, according to claim 1, wherein the protective screen (5) has a substantially annular shape, with a hole (10) that results arranged in axis with the opening (4) of the container (2).

3. A perfume device, according to claim 1 or 2, wherein the protective screen (5) is applied to the container (2) in a removable way.

4. A perfume device, according to one or more of claims from 1 to 3, wherein the container (2) is provided with a threaded neck (3) ending with said opening (4) and wherein the protective screen (5) forms a receiving channel (8) in which the neck (3) of the container (2) is inserted, the receiving channel (8) being provided with a thread (11) that is complementary to the thread of the neck (3) and of said hole (10) in such a way that the protective screen (5) results screwable on the neck (3) of the container with said openings (4, 10) in axis between them.

5. A perfume device, according to one or more of claims from 1 to 4, wherein the protective screen has a disk shape.

6. A perfume device, according to one or more of claims from 1 to 5, wherein the protective screen is concave for allowing the collection of the liquid.

7. A perfume device, according to one or more of claims from 1 to 6, wherein the protective screen is made of plastic or of a material permeable to liquids and covered at least partially with an impermeable layer.

8. A perfume device, according to one or more of claims from 1 to 7, wherein the protective screen includes an O-Ring (35) for retaining the collected liquid.

9. A perfume device, according to one or more of claims from 1 to 8, wherein the diffuser element (30) is arranged directly on the protective screen (5).

10. A perfume device, according to claim 9, wherein the diffuser element can be, according to the choice:
- Lain on the protective screen (5);
- Configured in such a way as to be fixed on the protective screen (5) with a pre-determined degree of mechanical interference.

11. A perfume device, according to one or more of the preceding claims, wherein a cage-shaped retain element (20) is included and is connected to the protective screen (5) so that the diffuser element is retained between the protective screen (5) and said cage (20).

12. A perfume device, according to claim 11, wherein the retain element (20) includes one or more hooks (21) at the ends that engage in a releasable manner into corresponding seats (13) found in the protective screen (5).

13. A perfume device, according to one or more of the preceding claims, wherein the perfume diffuser element (30) is entirely contained above the protective screen (5).

14. A perfume device, according to one or more of the preceding claims, wherein a pin (40) having a wide head (41) is included in such a way as to be blocked in the corresponding hole (10) of the protective screen (5), the pin being holed axially for the passage of the liquid contained in the container.

15. An assembly (5, 30) of perfume diffusion configured to result applicable in a removable way to a container (2) of perfuming liquid, said assembly comprising a protective screen (5) of annular shape, the protective screen being further provided with a hole (10) and being impermeable to liquids and above which a perfume diffuser element (30) is arranged, and c**haracterised in that** the perfume diffuser element (30) includes a blind channel (31), the diffuser element being further arranged with said channel (31) substantially in axis with the hole (10) in such a way that, in use, when the assembly is applied to the container (2), in correspondence of the overturning of the container (2) at least a part of the liquid contained in the container (2) is collected inside the channel (31).
